# EUROPEAN PATENT APPLICATION

(11) **EP 4 748 928 A1**
(43) Date of publication of application: **27.05.2026**
(21) Application number: 24843510.9
(22) Date of filing: 17.07.2024
(51) Int. Cl.: C12N 9/16, C12N 15/70, A23K 10/16

(54) **NOVEL ZEARALENONE-DEGRADING ENZYME AND USES THEREOF**

(30) Priority: 18.07.2023 KR 20230093242
(71) Applicant: CJ Cheiljedang Corporation, Seoul 04560 (KR)
(72) Inventor: LEE, Seung-hee, Seoul 04560 (KR); KIM, Nam Yoon, Seoul 04560 (KR); KANG, Young Seo, Seoul 04560 (KR); YANG, Tae Joo, Seoul 04560 (KR)
(74) Representative: Meissner Bolte Partnerschaft mbB
(86) International application number: PCT/KR2024/010300
(87) International publication number: WO 2025/018795

(57) **Abstract**

The present disclosure relates to a novel zearalenone-degrading enzyme and a use thereof.

## Description

### [Technical Field]

The present disclosure relates to a novel zearalenone-degrading enzyme and a use thereof.

### [Background Art]

Among the mycotoxins that contaminate cereals, zearalenone (ZEN) is a mycotoxin produced by fungi of the genus *Fusarium.* Zearalenone is a non-steroidal estrogenic mycotoxin, also referred to as F-2 mycotoxin or FES (fermentation estrogenic substance). It has a chemical structure and properties similar to estrogen, a type of female hormone, and can cause endocrine disruption in humans and livestock. For example, a recent study reported that receptors damaged by ZEN suppress estrogen hormones in mouse mammary tissue (Ecotoxicology and Environmental Safety Volume 241, August 2022, 113826). Further, it was reported that ZEN stimulates the growth of human breast cancer cells with estrogen-responsive receptors (Toxins 2019, 11(8), 481).

In particular, it is difficult to detect zearalenone poisoning at an early stage as zearalenone has weak acute toxicity and does not exhibit toxicity in a short period of time. Prolonged ingestion of cereals contaminated with zearalenone may induce infertility in pigs, and poisoning in humans and livestock can cause cancers or mutations. While zearalenone is frequently detected in cereals imported by South Korea, acute toxicity has not been reported; further, due to heat-stability, it can remain in processed foods and feeds made from contaminated cereals and cause harm by accumulating in humans and livestock.

Biological methods for removing zearalenone from cereals contaminated with mycotoxins include methods of using microorganisms to degrade the toxins. For example, WO 2006/053357 discloses fumonisin, fumonisin derivatives, and microorganisms exhibiting zearalenone-degrading activity, Korean Patent No. 1280811 discloses *Bacillus subtilis* GB-501 strain, a mutant strain exhibiting zearalenone-degrading activity, and Korean Patent No. 1494624 discloses *Agromyces* sp. M15 strain having zearalenone-degrading activity. However, since methods using microorganisms are merely additional effects resulting from substances secreted from the strains, they are limited in that the degradation activity of zearalenone itself is exhibited at a low level.

Accordingly, there is a need for an effective composition or method for effectively degrading and detoxifying zearalenone.

### [Disclosure]

### [Technical Problem]

A problem to be solved by the present disclosure is to provide a composition comprising one or more of a polypeptide of SEQ ID NO: 1, a polynucleotide encoding the polypeptide, a vector comprising the polynucleotide, and a host cell expressing the polypeptide of SEQ ID NO: 1; a use thereof; and a method for degrading zearalenone.

### [Technical Solution]

An object of the present disclosure is to provide a composition for degrading zearalenone, comprising one or more of a polypeptide having zearalenone-degrading activity, a polynucleotide encoding the polypeptide, a vector comprising the polynucleotide, and a host cell expressing the polypeptide.

Another object of the present disclosure is to provide a composition for detoxifying zearalenone present in a food, a feed, or both the food and the feed, comprising one or more of a polypeptide having zearalenone-degrading activity, a polynucleotide encoding the polypeptide, a vector comprising the polynucleotide, and a host cell expressing the polypeptide.

Yet another object of the present disclosure is to provide a feed additive composition, comprising one or more of a polypeptide having zearalenone-degrading activity, a polynucleotide encoding the polypeptide, a vector comprising the polynucleotide, and a host cell expressing the polypeptide.

Yet another object of the present disclosure is to provide a method for degrading zearalenone, comprising contacting one or more of a polypeptide having zearalenone-degrading activity and a host cell expressing the polypeptide with zearalenone.

Yet another object of the present disclosure is to provide a method for degrading zearalenone, comprising treating zearalenone with a composition comprising one or more of a polypeptide having zearalenone-degrading activity and a host cell expressing the polypeptide.

Yet another object of the present disclosure is to provide a method for detoxifying zearalenone, comprising treating a food or a feed comprising zearalenone with a composition comprising one or more of a polypeptide having zearalenone-degrading activity and a host cell expressing the polypeptide.

Yet another object of the present disclosure is to provide a method for preparing a polypeptide of SEQ ID NO: 1 having zearalenone-degrading activity, comprising culturing a host cell comprising one or more of a polypeptide having zearalenone-degrading activity; a polynucleotide encoding the polypeptide; and a vector comprising the polynucleotide.

Yet another object of the present disclosure is to provide a use of a polypeptide of SEQ ID NO: 1 as a zearalenone-degrading enzyme.

Yet another object of the present disclosure is to provide a use of a composition comprising one or more of a polypeptide having zearalenone-degrading activity, a polynucleotide encoding the polypeptide, a vector comprising the polynucleotide, and a host cell expressing the polypeptide, for degrading zearalenone.

### [Advantageous Effects]

Zearalenone, a mycotoxin, can be effectively degraded using the polypeptide having zearalenone-degrading activity of the present disclosure.

### [Brief Description of the Drawings]

FIG. 1 shows the confirmation of activity of the novel zearalenone-degrading enzyme of the present disclosure by HPLC-UV.
FIG. 2 shows the comparison of activity between the novel zearalenone-degrading enzyme of the present disclosure and an α/β hydrolase enzyme by HPLC-UV.

### [Detailed Description of Preferred Embodiments]

The present disclosure will be described in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below.

In addition, those skilled in the art will recognize, or be able to ascertain using no more than routine experimentation, many equivalents to the specific aspects described in the present disclosure. In addition, such equivalents are intended to be included in the present disclosure.

Furthermore, a number of papers and patent documents are referenced and cited throughout the present specification. The disclosures of the cited papers and patent documents are incorporated herein by reference in their entirety to further clarify the level of art and the description of the present disclosure.

One aspect of the present disclosure is a polypeptide of SEQ ID NO: 1 having zearalenone-degrading activity. The polypeptide may also be referred to as "zearalenone-degrading enzyme".

Another aspect of the present disclosure is a use of a polypeptide of SEQ ID NO: 1 as a zearalenone-degrading enzyme.

Another aspect of the present disclosure is a composition for degrading zearalenone, comprising one or more of a polypeptide having zearalenone-degrading activity, a polynucleotide encoding the polypeptide, a vector comprising the polynucleotide, and a host cell expressing the polypeptide.

The expression "degradation of zearalenone" may be used interchangeably with "detoxification of zearalenone", "inactivation of zearalenone", and "decontamination of zearalenone".

The term "zearalenone (ZEN)" in the present disclosure refers to a non-steroidal estrogenic mycotoxin produced by fungi of the genus *Fusarium,* particularly by strains of *F*. *graminearum* (Gibberella zeae), *F*. *culmorum, F*. *cerealis, F*. *equiseti, F*. *crookwellense, F*. *semitectum, etc.,* and is known as one of the mycotoxins widely distributed in cereals worldwide. The zearalenone is also referred to as F-2 toxin, Fusarium toxin, and FES (fermentation estrogenic substance) and has about 20 types of isomers, but trans-α-zearalenone mainly occurs in cereals in nature. Zearalenone has been reported to cause damage through actions similar to sex hormones rather than toxicity. Most cases of poisoning are hyperhormonal but can also cause cancer and mutations. For example, zearalenone induces infertility in pregnant sows in the livestock industry; ovarian abnormalities, premature birth, miscarriage, *etc.* have been reported; and even a concentration of 50 ppb in feed is known to cause changes in pig ovaries.

In one example, zearalenone-degrading activity can be confirmed by detecting the residual amount of zearalenone.

Meanwhile, although the polypeptide having zearalenone-degrading activity provided by the present disclosure has been defined as the polypeptide of SEQ ID NO: 1, this does not exclude the addition of nonfunctional sequences upstream or downstream of the amino acid sequence of SEQ ID NO: 1, naturally occurring mutations, silent mutations thereof, or conservative substitutions. Further, it is apparent to those skilled in the art that any protein that exhibits an activity identical or corresponding to that of the protein composed of SEQ ID NO: 1 corresponds to the polypeptide having zearalenone-degrading activity provided by the present disclosure.

That is, even if the present disclosure describes a "protein or a polypeptide of an amino acid sequence represented by a specific SEQ ID NO", a "protein or a polypeptide having an amino acid sequence represented by a specific SEQ ID NO", or a "protein or a polypeptide comprising an amino acid sequence represented by a specific SEQ ID NO", it is apparent that a protein having an amino acid sequence with deletion, modification, substitution, or addition in part of the sequence may also be used in the present disclosure, as long as it has an activity identical or corresponding to that of the polypeptide consisting of the amino acid sequence of the corresponding SEQ ID NO.

Further, variant polypeptides that differ from the recited sequence in one or more amino acids by conservative substitution and/or modification compared to the amino acid sequence of SEQ ID NO: 1, but retain the functions or properties of the protein, are also included in the scope of polypeptides provided by the present disclosure. Such modifications may include, for example, a modification in which a portion is removed from the N- and/or C-terminus of the mature protein.

The term "conservative substitution" in the present disclosure means the substitution of one amino acid with another amino acid having similar structural and/or chemical properties. The polypeptide of the present disclosure may have, for example, one or more conservative substitutions while still retaining one or more biological activities of the polypeptide of SEQ ID NO: 1. Such an amino acid substitution may generally occur based on similarity in the polarity, charge, solubility, hydrophobicity, hydrophilicity, and/or amphipathic nature of residues.

In one embodiment, the polypeptide provided by the present disclosure may include a polypeptide that consists of, comprises, or essentially consists of SEQ ID NO: 1 or an amino acid sequence having at least 80% or more, for example at least 85%, 90%, 95%, 97%, or 99% or more homology or identity thereto. Further, a polypeptide having the homology or identity and zearalenone-degrading activity is included in the polypeptide having zearalenone-degrading activity provided by the present disclosure.

In one embodiment, the polypeptide provided by the present disclosure may be derived from a *Sphingomonas* sp. microorganism. In one embodiment, the zearalenone-degrading enzyme of the present disclosure may be a polypeptide derived from the microorganism having α/β hydrolase activity, but is not limited thereto.

As used herein, the term "homology" or "identity" refers to the degree of relatedness between two given amino acid sequences or nucleotide sequences and may be expressed as a percentage. The terms "homology" and "identity" may often be used interchangeably.

The sequence homology or identity of a conserved polynucleotide or polypeptide is determined by standard alignment algorithms, and a default gap penalty established by the program used may be used together. Substantially, homologous or identical sequences are generally capable of hybridizing with the whole sequence or a part of the sequence, corresponding to at least about 50%, 60%, 70%, 80%, or 90% of the full length, under moderately or highly stringent conditions. It is apparent that hybridization also includes a polynucleotide containing a general codon or a codon considering codon degeneracy in the polynucleotide.

Whether any two polynucleotide or polypeptide (including protein) sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.] (1988) SIAM J Applied Math 48: 1073)). For example, homology, similarity, or identity may be determined using BLAST of the National Center for Biotechnology Information, or ClustalW.

The homology, similarity, or identity between polynucleotides or polypeptides (including proteins) may be determined by, for example, comparing sequence information using a GAP computer program, such as Needleman et al., (1970), J Mol Biol. 48:443, as described in, for example, Smith and Waterman, Adv. Appl. Math (1981) 2:482. Briefly, a GAP program may be defined as the value acquired by dividing the number of similarly aligned symbols (namely, nucleotides or amino acids) by the total number of symbols in the shorter of two sequences. The default parameters for the GAP program may include: (1) a binary comparison matrix (comprising values of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

Further, whether any two polynucleotide or polypeptide (including protein) sequences have homology, similarity, or identity may be confirmed by comparing sequences through Southern hybridization experiments under defined stringent conditions, and the defined appropriate hybridization conditions are within the scope of the technical field and can be determined by methods well known to those skilled in the art (such as J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York).

As used herein, the term "polynucleotide", which is a polymer of nucleotides in which nucleotide monomers are connected in a long chain shape by covalent bonds, refers to a DNA or RNA strand having a certain length or longer.

The polynucleotide encoding the polypeptide having zearalenone-degrading activity of the present disclosure may include, without limitation, a polynucleotide encoding the polypeptide of SEQ ID NO: 1 and a polynucleotide encoding a polypeptide having an activity corresponding thereto. For example, the polynucleotide encoding the polypeptide having zearalenone-degrading activity of the present disclosure may be a polynucleotide sequence encoding the amino acid sequence of SEQ ID NO: 1, or a polynucleotide sequence encoding a polypeptide having at least 80% or more homology or identity thereto.

In the polynucleotide encoding the polypeptide having zearalenone-degrading activity of the present disclosure, various modifications may be made in the coding region as long as the amino acid sequence of the polypeptide is not changed in consideration of codon degeneracy or codons preferred in organisms that are intended to express the polypeptide.

In one embodiment, the polynucleotide encoding the polypeptide having zearalenone-degrading activity of the present disclosure may consist of or essentially consist of a nucleotide sequence having at least 70%, 75%, 80%, 85%, 90%, 95%, 96%, 97%, 98%, or 99% or more homology or identity to SEQ ID NO: 2, but is not limited thereto. Further, the polynucleotide of the present disclosure may comprise a probe that can be prepared from a known gene sequence, for example, without limitation as long as it is a sequence capable of hybridizing with a complementary sequence to the entirety or part of the polynucleotide sequence of the present disclosure under stringent conditions.

The term "stringent conditions" refers to conditions that enable specific hybridization between polynucleotides. These conditions are specifically described in the literature (see J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York, 9.50-9.51, 11.7-11.8). Examples of stringent conditions may be conditions in which polynucleotides having high homology or identity of 70% or more, 75% or more, 80% or more, 85% or more, 90% or more, 95% or more, 96% or more, 97% or more, 98% or more, or 99% or more, hybridize with each other, but polynucleotides having low homology or identity do not hybridize with each other; or washing conditions of typical Southern hybridization, i.e., conditions of washing once, or specifically two to three times, at a salt concentration and temperature corresponding to 1×SSC, 0.1% SDS at 60°C, specifically 0.1×SSC, 0.1% SDS at 60°C, or more specifically 0.1×SSC, 0.1% SDS at 68°C.

Hybridization requires that two nucleic acids have complementary sequences, although mismatches between bases may be possible depending on the stringency of hybridization. The term "complementary" is used to describe the relationship between nucleotide bases capable of hybridizing with each other. For example, with regard to DNA, adenine is complementary to thymine, and cytosine is complementary to guanine. Hence, the polynucleotide of the present disclosure may also include substantially similar nucleic acid sequences as well as isolated nucleic acid fragments that are complementary to the entire sequence.

Specifically, polynucleotides having homology or identity to the polynucleotide of the present disclosure may be detected by employing hybridization conditions comprising a hybridization step at a Tm of 55°C and utilizing the above-described conditions. In addition, the Tm value may be 60°C, 63°C, or 65°C, but is not limited thereto, and may be appropriately adjusted by those skilled in the art.

The appropriate stringency for hybridizing the polynucleotide depends on the length and degree of complementarity of the polynucleotide, and the variables are well known in the relevant technical field (such as J. Sambrook *et al., supra*)*.*

In the present disclosure, the term "vector" refers to a DNAconstruct containing a base sequence of a polynucleotide encoding the desired polypeptide that is operably linked to a suitable expression regulatory region (or expression control sequence) such that the desired polypeptide may be expressed in a suitable host. The expression regulatory region may comprise a promoter capable of initiating transcription, any operator sequence for controlling such transcription, a sequence encoding a suitable mRNA ribosome binding site, and a sequence controlling termination of transcription and translation. The vector, after being transformed into a suitable host cell, may replicate or function independently of the host genome, or may be integrated into the genome itself.

For example, a polynucleotide encoding a target protein can be expressed in a chromosome through a vector for chromosomal insertion in a cell. The insertion of the polynucleotide into the chromosome may be performed by any method known in the art, for example, homologous recombination, but is not limited thereto. It may further comprise a selection marker for confirming the insertion into the chromosome. The selection marker is for selecting cells transformed with vectors, that is, for confirming the insertion of a desired nucleic acid molecule, and markers that confer selectable phenotypes such as drug resistance, auxotrophy, resistance to cytotoxic agents, or expression of surface proteins may be used. In an environment treated with a selective agent, only cells expressing the selection marker survive or exhibit other phenotypic traits, thereby enabling selection of transformed cells.

The vector used in the present disclosure is not particularly limited, and any vector known in the art may be used.

Examples of commonly used vectors in prokaryotic cells, pWE15, M13, MBL3, MBL4, IXII, ASHII, APII, t10, t11, Charon4A, Charon21A, *etc.* may be used as a phage vector or a cosmid vector, and pDZ system, pDC system, pBR system, pUC system, pBluescript II system, pGEM system, pTZ system, pCL system, pET system, *etc.* may be used as a plasmid vector. Specifically, pDZ, pDC, pDCM2, pACYC177, pACYC184, pCL, pECCG117, pUC19, pBR322, pMW118, pCC1BAC vectors, *etc*. may be used.

As an example of a vector used in eukaryotic cells, a yeast expression vector may be both an integrative yeast plasmid (Ylp) or an extrachromosomal plasmid vector. The extrachromosomal plasmid vector may include episomal yeast plasmid (YEp), replicative yeast plasmid (YRp), and yeast centromer plasmid (YCp). In addition, artificial yeast chromosomes (YACs) may also be used as the vector of the present disclosure. As specific examples, vectors that can be used include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405, and pYJ406, but are not limited thereto.

As used herein, the term "transformation" refers to the introduction of a vector comprising a polynucleotide encoding the desired protein into a host cell or a microorganism, such that the protein encoded by the polynucleotide can be expressed in the host cell. The transformed polynucleotide may include both a polynucleotide inserted and located within a chromosome of the host cell and a polynucleotide located outside the chromosome, as long as the protein encoded by the polynucleotide can be expressed in the host cell. Further, the polynucleotide comprises a DNA and/or RNA encoding the desired protein. The polynucleotide may be introduced into a host cell in any form, as long as it can be expressed therein. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replication. Further, the polynucleotide may be introduced into a host cell in the form *per se* and be operably linked to a sequence required for expression in the host cell, but is not limited thereto.

In addition, as used herein, the term "operably linked" means that a polynucleotide encoding the desired polypeptide of the present disclosure is functionally linked to a promoter sequence that initiates and mediates transcription of the polynucleotide.

The method of transforming a vector of the present disclosure comprises any method of introducing a nucleic acid into a cell, and may be performed by selecting a suitable standard technique known in the art depending on the host cell. For example, methods such as electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method may be used, but the method is not limited thereto.

The host cell expressing the polypeptide having zearalenone-degrading activity of the present disclosure may be a host cell comprising one or more of the polypeptide having zearalenone-degrading activity of the present disclosure; a polynucleotide encoding the polypeptide; and a vector comprising the polynucleotide.

In one embodiment, the vector may, as described above, be integrated into a chromosome or be maintained as a self-replicating extrachromosomal vector.

The host cell of the present disclosure may be any cell useful for recombinant production of a polypeptide having zearalenone-degrading activity, for example, a prokaryotic cell or a eukaryotic cell. In one example, the host cell may be a fungal cell. In one example, the prokaryotic host cell may be any gram-positive or gram-negative bacteria.

In one example, the host cell may be a microorganism. For instance, the host cell may be *Escherichia coli* (*E. coli*), but is not limited thereto.

The polypeptide having zearalenone-degrading activity of the present disclosure; a polynucleotide encoding the polypeptide; a vector comprising the polynucleotide; and/or a host cell expressing the polypeptide having zearalenone-degrading activity may be used for degrading zearalenone.

In one embodiment, the zearalenone may be present in a food.

In one embodiment, the zearalenone may be present in a feed.

The composition of the present disclosure may be used to degrade and detoxify zearalenone present in a food and/or a feed.

Another aspect of the present disclosure is a composition for detoxifying zearalenone present in a food, a feed, or both the food and the feed, comprising one or more of the polypeptide having zearalenone-degrading activity of the present disclosure; a polynucleotide encoding the polypeptide; a vector comprising the polynucleotide; and a host cell expressing the polypeptide having zearalenone-degrading activity.

Another aspect of the present disclosure is a feed additive composition comprising one or more of the polypeptide having zearalenone-degrading activity of the present disclosure; a polynucleotide encoding the polypeptide; a vector comprising the polynucleotide; and a host cell expressing the polypeptide having zearalenone-degrading activity.

In one embodiment, the composition of the present disclosure may further comprise a naturally occurring substance or a non-naturally occurring substance.

Examples of substances that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, isotonic agents, diluents, lubricants, *etc.,* but are not limited thereto.

Another aspect of the present disclosure is a method for degrading zearalenone, comprising contacting the polypeptide having zearalenone-degrading activity of the present disclosure and/or a host cell expressing the polypeptide having zearalenone-degrading activity with zearalenone.

The polypeptide having zearalenone-degrading activity and a host cell expressing the same are as described above.

Another aspect of the present disclosure is a method for degrading zearalenone, comprising treating zearalenone with a composition comprising one or more of the polypeptide having zearalenone-degrading activity of the present disclosure and a host cell expressing the polypeptide having zearalenone-degrading activity.

The polypeptide having zearalenone-degrading activity and a host cell expressing the same are as described above.

Another aspect of the present disclosure is a method for detoxifying zearalenone, comprising treating a food or a feed comprising zearalenone with a composition comprising one or more of the polypeptide having zearalenone-degrading activity of the present disclosure and a host cell expressing the polypeptide having zearalenone-degrading activity.

The polypeptide having zearalenone-degrading activity and a host cell expressing the same are as described above.

In the composition and method of the present disclosure, a polypeptide having zearalenone-degrading activity may be recovered from the microorganism expressing the polypeptide, or without recovery, the host cell expressing the polypeptide *per se* may be used as a source of the polypeptide.

Another aspect of the present disclosure is a method for preparing a polypeptide of SEQ ID NO: 1 having zearalenone-degrading activity, comprising culturing a host cell comprising one or more of the polypeptide having zearalenone-degrading activity of the present disclosure; a polynucleotide encoding the polypeptide; and a vector comprising the polynucleotide.

The polypeptide having zearalenone-degrading activity and a host cell expressing the same are as described above.

Another aspect of the present disclosure is to provide a use of a polypeptide of SEQ ID NO: 1 as a zearalenone-degrading enzyme.

The polypeptide of SEQ ID NO: 1 is as described above.

Another aspect of the present disclosure is to provide a use of a composition comprising one or more of the polypeptide having zearalenone-degrading activity of the present disclosure, a polynucleotide encoding the polypeptide, a vector comprising the polynucleotide, and a host cell expressing the polypeptide, for degrading zearalenone.

The polypeptide having zearalenone-degrading activity and a host cell expressing the same are as described above.

### [Mode for Carrying Out the Invention]

Hereinafter, the present disclosure will be described in more detail by way of Examples and Experimental Examples. However, the following Examples and Experimental Examples are only for illustrative purposes of the present disclosure, and thus the scope of the present disclosure is not limited thereto.

### Example 1: Preparation of Expression Vector for Novel ZEN-Degrading Enzyme ZOR 7

A polynucleotide (SEQ ID NO:2) encoding the α/β hydrolase derived from *Sphingomonas* sp. (hereinafter referred to as ZOR_7, SEQ ID NO:1)was synthesized by Cosmo Genetech and cloned into a pET vector (Novagen) using the primers shown in Table 1.

**[Table 1]**

| | SEQ ID NO | Name | Primer Sequence (5'- 3') |
|---|---|---|---|
| ZOR_7 | 3 | ZOR-7-F | gatataccatggggATGACGAGCGAAACCGGCG |
| | 4 | ZOR-7-R | cgagtgcggccgcCGCATCCGGCAGGGTCG |

Specifically, ZOR_7 was synthesized by PCR using a gene construct, primers (SEQ ID NOs: 3 and 4 in Table 1), and a PCR premix (iNtRON, cat. no. 25185). PCR was performed using an Eppendorf Mastercycler Nexus GX2, and the reaction conditions were as follows:
- Initial denaturation - 94°C, 2 min
- Denaturation - 94°C, 20 sec
- Annealing - 55°C, 10 sec
- Extension - 72°C, 2 min (25 cycles of denaturation to extension)
- Final Extension - 72°C, 5 min

The PCR product and the vector obtained were treated with restriction enzymes (Ncol and Notl) followed by ligation using T4 DNA ligase (NEB, Cata# M0202S), which was then transformed into *E. coli* DH5α strain and verified by sequencing to confirm the absence of any sequence variation.

### Example 2: Expression and Purification of Novel ZEN-Degrading Enzyme ZOR 7 for Activity Evaluation

ZOR_7 prepared in Example 1 was transformed into *E. coli* BL21 (DE3), and inoculated into sterilized LB medium (BD Difco), followed by pre-culture at 37°C and 200 rpm for 16 hours. Afterwards, 1/100 of the medium volume was inoculated into a flask containing sterilized LB medium and cultured at 37°C and 200 rpm until absorbance (OD₆₀₀) of between 0.4 and 0.5. Then, isopropyl β-D-1-thiogalactopyranoside (IPTG) was added to a final concentration of 1 mM and the cells were cultured for an additional 16 hours, after which the cells were recovered by centrifugation. The recovered cells were resuspended in 20 ml of lysis buffer (50 mM Tris-HCl pH 8.0, 100 mM NaCl, 10 mM imidazole), and the crude enzyme extract was obtained through sonication and centrifugation. The crude enzyme extract was adsorbed onto Ni-NTA resin (Qiagen, Cat no. 30230), then the enzyme was purified by sequentially flowing the washing buffer (lysis buffer composition above, with 20 mM imidazole) and the elution buffer (lysis buffer composition above, with 250 mM imidazole).

Protein concentration was determined by mixing 5 µL of diluted enzyme solution and 250 µL of Bradford solution (Quick Start^{™} Bradford 1x Dye Reagent, #5000205) and measuring the absorbance at 595 nm.

### Example 3: Activity Evaluation Using HPLC-UV

To analyze the activity of ZOR_7 prepared in Example 2 against ZEN, the enzyme reaction solution (50 mM Tris HCl, 150 mM NaCl, pH 7.4) purified in Example 2 was treated with zearalenone (CAS 17924-92-4) dissolved in acetonitrile solution. The reaction was performed for 3 hours at 37°C, then stopped by treating with methanol.

The residual amount of ZEN in the reaction product was measured using High performance liquid chromatography-Ultraviolet (HPLC-UV). The detailed analysis conditions are as follows:
(1) Chromatography: Agilent Technologies 1260 Infinity II HPLC System
(2) Column: Inertsil ODS-3 C18 5um 4.6x250mm
(3) Column temperature: 30°C
(4) Flow rate: 1 mL/min
(5) Sample injection: 20.0 µL
(6) Mobile phase: A: Water, B: Acetonitrile
(7) Elution conditions: Isocratic elution (40% water/60% Acetonitrile), detection wavelength 272 nm

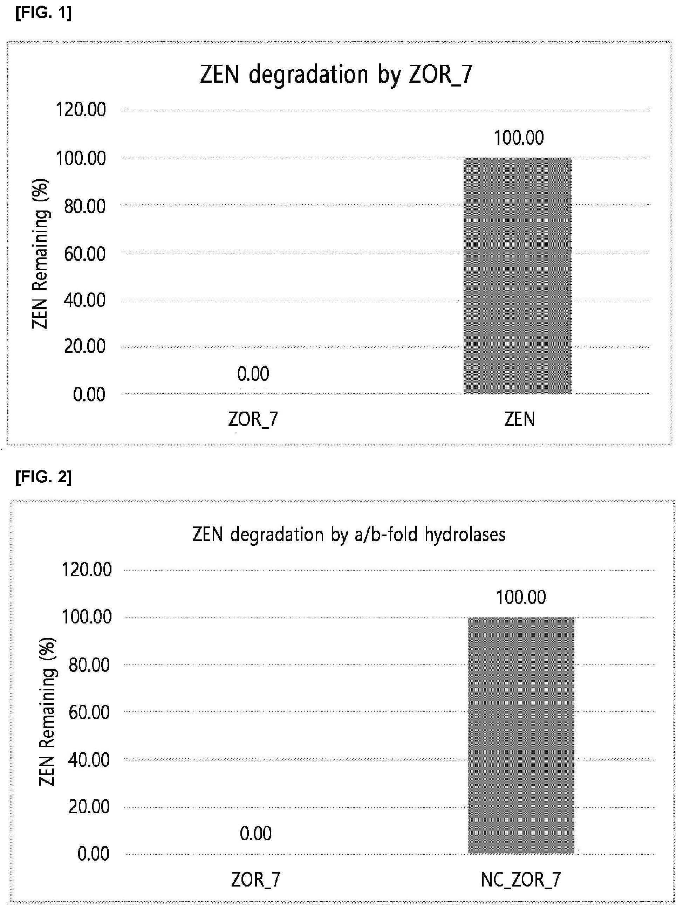

It was observed that ZOR_7 completely degrades ZEN. The detailed results are as shown in FIG. 1.

Accordingly, it was confirmed that ZOR_7 of the present disclosure has zearalenone-degrading ability.

### Example 4: Comparison of Activity between Novel ZEN-Degrading Enzyme ZOR 7 and α/β Hydrolase Enzyme

### Example 4-1: Preparation of Expression Vector for Control α/β Hydrolase Enzyme derived from Sphingomonas sp.

A polynucleotide (SEQ ID NO: 6) encoding the control α/β hydrolase derived from *Sphingomonas* sp. (hereinafter referred to as NC_ZOR_7, SEQ ID NO:5) was synthesized by Cosmo Genetech and cloned into a pET vector (Novagen) using the primers shown in Table 2.

**[Table 2]**

| | SEQ ID NO | Name | Primer Sequence (5'- 3') |
|---|---|---|---|
| NC_ZOR_7 | 7 | NC_ZOR_7_F | gatataccatggggATGAAGTTGCGCCTGA CGATATTG |
| | 8 | NC_ZOR_7_R | ctcgagtgcggccgcTTTGCGACCATGTCCT AAATAACG |

Specifically, ZOR_7 was synthesized by PCR using a gene construct, primers (SEQ ID NOs: 7 and 8 in Table 2), and a PCR premix (iNtRON, cat. no. 25185). PCR was performed using an Eppendorf Mastercycler Nexus GX2, and the reaction conditions were as follows:
- Initial denaturation - 94°C, 2 min
- Denaturation - 94°C, 20 sec
- Annealing - 55°C, 10 sec
- Extension - 72°C, 2 min (25 cycles of denaturation to extension)
- Final Extension - 72°C, 5 min

The PCR product and the vector obtained were treated with restriction enzymes (Ncol and Notl) followed by ligation using T4 DNA ligase (NEB, Cata# M0202S), which was then transformed into *E. coli* DH5α strain and verified by sequencing to confirm the absence of any sequence variation.

### Example 4-2: Expression and Purification of Control α/β Hydrolase Enzyme derived from Sphingomonas sp.

NC_ZOR_7 prepared in Example 4-1 was transformed into *E. coli* BL21 (DE3), and inoculated into sterilized LB medium (BD Difco), followed by pre-culture at 37°C and 200 rpm for 16 hours. Afterwards, 1/100 of the medium volume was inoculated into a flask containing sterilized LB medium and cultured at 37°C and 200 rpm until absorbance (OD₆₀₀) of between 0.4 and 0.5. Then, isopropyl β-D-1-thiogalactopyranoside (IPTG) was added to a final concentration of 1 mM and the cells were cultured for an additional 16 hours, after which the cells were recovered by centrifugation. The recovered cells were resuspended in 20 ml of lysis buffer (50 mM Tris-HCI pH 8.0, 100 mM NaCl, 10 mM imidazole), and the crude enzyme extract was obtained through sonication and centrifugation. The crude enzyme extract was adsorbed onto Ni-NTA resin (Qiagen, Cat no. 30230), then the enzyme was purified by sequentially flowing the washing buffer (lysis buffer composition above, with 20 mM imidazole) and the elution buffer (lysis buffer composition above, with 250 mM imidazole).

Protein concentration was determined by mixing 5 µL of diluted enzyme solution and 250 µL of Bradford solution (Quick Start^{™} Bradford 1x Dye Reagent, #5000205) and measuring the absorbance at 595 nm.

### Example 4-3: Activity Evaluation Using HPLC-UV

To comparatively analyze the activity of ZOR_7 prepared in Example 2 and the control NC_ZOR_7 produced in Example 4-2 against ZEN, each enzyme reaction solution (50 mM Tris HCl, 150 mM NaCl, pH 7.4) purified in Example 2 or Example 4-2 was treated with zearalenone (CAS 17924-92-4) dissolved in acetonitrile solution. The reaction was performed for 3 hours at 37°C, then stopped by treating with methanol.

The residual amount of ZEN in the reaction product was measured using High performance liquid chromatography-Ultraviolet (HPLC-UV). The detailed analysis conditions are as follows:
(1) Chromatography: Agilent Technologies 1260 Infinity II HPLC System
(2) Column: Inertsil ODS-3 C18 5um 4.6x250mm
(3) Column temperature: 30°C
(4) Flow rate: 1 mL/min
(5) Sample injection: 20.0 µL
(6) Mobile phase: A: Water, B: Acetonitrile
(7) Elution conditions: Isocratic elution (40% water/60% Acetonitrile), detection wavelength 272 nm

It was observed that while ZOR_7 completely degrades ZEN, the control NC_ZOR_7 does not degrade ZEN. The detailed results are as shown in FIG. 2.

Accordingly, it was confirmed that not all α/β hydrolases derived from *Sphingomonas* sp. have ZEN-degrading ability, and even amongst α/β hydrolases derived from *Sphingomonas sp,* only a specific enzyme, namely ZOR_7 of the present disclosure, possesses unique zearalenone-degrading ability.

From the foregoing description, those skilled in the art to which the present disclosure pertains will be able to understand that the present disclosure may be embodied in other specific forms without modifying the technical concepts or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts fall within the scope of the present disclosure.

## Claims

1. A composition for degrading zearalenone, comprising one or more of:
a polypeptide of SEQ ID NO: 1;
a polynucleotide encoding the polypeptide;
a vector comprising the polynucleotide; and
a host cell expressing the polypeptide of SEQ ID NO: 1.

2. A composition for detoxifying zearalenone present in a food, a feed, or both the food and the feed, comprising one or more of:
a polypeptide of SEQ ID NO: 1;
a polynucleotide encoding the polypeptide;
a vector comprising the polynucleotide; and
a host cell expressing the polypeptide of SEQ ID NO: 1.

3. A feed additive composition, comprising one or more of:
a polypeptide of SEQ ID NO: 1;
a polynucleotide encoding the polypeptide;
a vector comprising the polynucleotide; and
a host cell expressing the polypeptide of SEQ ID NO: 1.

4. A method for degrading zearalenone, comprising contacting one or more of:
a polypeptide of SEQ ID NO: 1;
a polynucleotide encoding the polypeptide;
a vector comprising the polynucleotide; and
a host cell expressing the polypeptide of SEQ ID NO: 1 with zearalenone.

5. A method for preparing a polypeptide of SEQ ID NO: 1 having zearalenone-degrading activity, comprising culturing a host cell comprising one or more of a polypeptide of SEQ ID NO: 1; a polynucleotide encoding the polypeptide; and a vector comprising the polynucleotide.

6. Use of a composition comprising one or more of a polypeptide of SEQ ID NO: 1; a polynucleotide encoding the polypeptide; a vector comprising the polynucleotide; and a host cell expressing the polypeptide of SEQ ID NO: 1, for degrading zearalenone.
